# DEMANDE DE BREVET EUROPEEN

(11) **EP 3 922 222 A1**
(43) Date de publication de la demande: **15.12.2021**
(21) Numéro de dépôt: 21178271.9
(22) Date de dépôt: 08.06.2021
(51) Int. Cl.: A61F 5/01

(54) **ORTHÈSE DYNAMIQUE**

(30) Priorité: 09.06.2020 FR 2006018
(71) Demandeur: Texssedre, Hervé, 8125-403 Vilamoura (PT)
(72) Inventeur: Texssedre, Hervé, 8125-403 Vilamoura (PT)
(74) Mandataire: Camus, Olivier Jean-Claude

(57) **Abrégé**

Un aspect de l'invention concerne une orthèse dynamique (100) conçue pour être montée sur une chaussure ou une semelle logée à l'intérieur d'une chaussure, soutenant le pied d'un patient et comportant :
- une embase de jambe (110) adaptée pour entourer la jambe du patient, et
- une structure de liaison mobile (130), reliant la chaussure et l'embase et comportant un vérin élastique (131) déporté sur l'arrière de la jambe du patient, une extension axiale (132) dudit vérin et une articulation (133) reliant l'extension axiale à la chaussure,
l'embase (110) comportant une coque (111) apte à emboiter une partie de la jambe du patient et un système d'attache réglable (150) apte à maintenir la coque autour de ladite jambe à une distance prédéfinie de la chaussure.

## Description

### DOMAINE TECHNIQUE DE L'INVENTION

La présente invention concerne une orthèse dynamique de pied adaptée à l'aide à la marche ou à la rééducation d'un patient atteint d'une paralysie du pied ou bien des muscles ou nerfs releveurs.

L'invention trouve des applications dans le domaine médical et, en particulier, dans le domaine de l'orthopédie.

### ARRIERE-PLAN TECHNOLOGIQUE DE L'INVENTION

Dans le domaine médical, les patients atteints de certaines affections musculaires ou neurologiques, telles que l'hémiplégie, la sclérose en plaques, les polynévrites, etc., peuvent avoir un pied ballant, c'est-à-dire que leur pointe de pied tombe naturellement vers le sol lorsque la jambe est relevée. Cet effet de pied ballant, appelé steppage du pied, est la conséquence d'une absence partielle ou totale de motricité musculaire rendant l'articulation de la cheville molle. Un patient souffrant d'un steppage du pied doit, pour se déplacer, lever exagérément la jambe de façon à ce que son pied n'accroche pas le sol.

Pour maintenir le pied du patient dans une position non-ballante, il est connu d'utiliser des orthèses rigides ou semi-rigides qui bloquent le pied dans une position sensiblement perpendiculaire à la jambe. Ces orthèses empêchent le pied de tomber mais bloquent également tous les mouvements de flexion du pied. Or, ces mouvements de flexion sont nécessaires à la marche. En conséquence, un patient équipé d'une orthèse rigide ou semi-rigide a une démarche asymétrique, de type claudication. En outre, de telles orthèses ne participent pas au travail de rééducation du pied du patient puisque le pied est totalement bloqué dans l'orthèse. Or, il est reconnu, dans le domaine médical, qu'il est important de faire travailler un membre, même lorsqu'il est paralysé, afin d'entretenir les muscles et articulations qui ne sont pas endommagées.

Il existe un autre type d'orthèse, moins rigide, qui permet la rééducation fonctionnelle et l'entretien des muscles et articulations, et en particulier des muscles releveurs. Cette orthèse - appelée orthèse dynamique - comporte un moyen pour repousser le talon lorsque le pied est ballant, ou pour le tirer vers la jambe lorsque le pied est en appui. Une telle orthèse coopère avec le pied du patient, notamment avec l'articulation de la cheville et les articulations antérieures du pied. Il n'est donc pas nécessaire d'utiliser un organe externe d'articulation du pied, ce qui limite l'encombrement généré par l'orthèse.

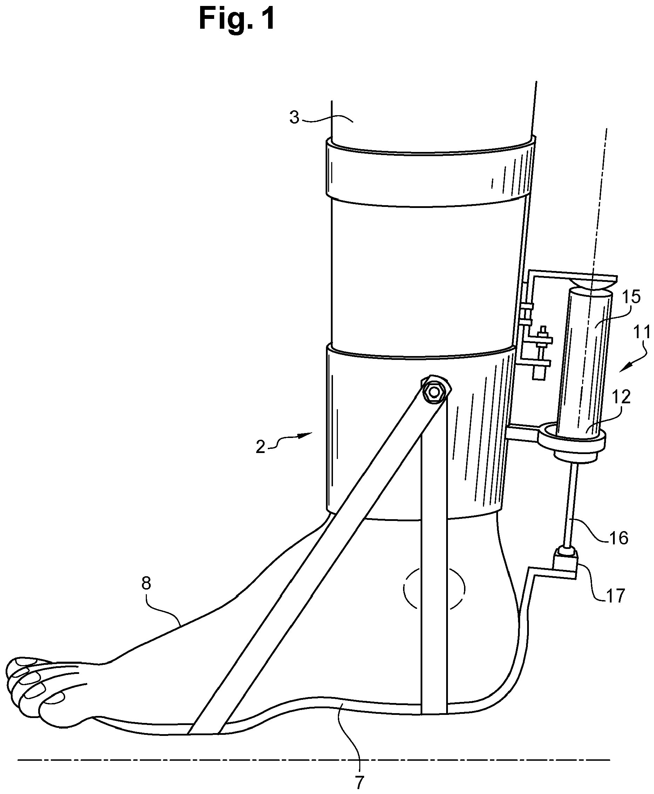

Un exemple d'une telle orthèse est représenté sur la figure 1. Cette orthèse comporte un bracelet de jambe 2 et une semelle de pied 7 reliés ensemble par une structure de liaison 11. La structure de liaison 11 comporte un vérin 12 déporté sur l'arrière de la jambe 3 et du pied 8. Le vérin 12 est extensible longitudinalement pour faire pivoter la semelle 7 autour de l'articulation de la cheville. Une telle orthèse est décrite notamment dans les brevets EP 2 506 811 et EP 1 382 317, au nom du demandeur.

Le vérin 12 est un vérin élastique qui comporte une extension axiale 16, 17. Cette extension axiale est généralement une tige mobile 16 montée dans un corps de vérin 15. Une extrémité inférieure de la tige de vérin 16 est, par exemple, reliée à la semelle de pied 7 au niveau du talon, tandis que le corps de vérin 15 est relié au bracelet de jambe 2. Le vérin peut par exemple être un vérin pneumatique ou hydropneumatique. Il peut également s'agir d'un vérin mécanique, comportant un ressort hélicoïdal à l'intérieur du corps de vérin.

Le vérin 12 est réglé de sorte que, lorsque le pied du patient est sensiblement perpendiculaire à l'orientation de la jambe, le vérin se trouve en semi-compression. Lorsque le patient soulève sa jambe du sol, la tige 16 du vérin se déplace vers le bas, entraînant le talon de la semelle de pied 7. La semelle pivote, déplaçant vers le haut la partie antérieure du pied du patient, ce qui empêche les orteils dudit pied de frotter sur le sol au cours de la marche. Lorsque le patient pose à nouveau son talon sur le sol, la tige du vérin est poussée vers le haut. La partie antérieure du pied du patient pivote vers le bas et entre en contact avec le sol.

Une telle orthèse nécessite une fabrication unitaire, c'est-à-dire qu'elle doit être fabriquée spécifiquement pour chaque patient. En effet, les mouvements du pied étant contrôlés par le vérin, le positionnement du vérin doit être adapté à la longueur de la jambe et la force du vérin doit être adaptée à la pathologie du patient afin que le contrôle soit approprié au patient. De plus, le bracelet étant rigide, il doit être adapté aux dimensions de la jambe du patient afin d'être correctement maintenu autour de sa jambe sans toutefois compresser et blesser la jambe. La fabrication de l'orthèse étant spécifique à chaque patient, le patient est contraint de rencontrer une première fois le thérapeute, ou le fabriquant, pour déterminer les dimensions du bracelet et le positionnement du vérin et une seconde fois pour recevoir l'orthèse, un temps d'attente étant nécessaire entre les deux rencontres pour fabriquer l'orthèse.

Il existe donc un réel besoin d'une orthèse générique pouvant facilement être adaptée à chaque patient.

### RESUME DE L'INVENTION

Pour répondre aux problèmes évoqués ci-dessus de fabrication spécifique des orthèses, le demandeur propose une orthèse dynamique, générique, comportant une coque et un système d'attache de la coque ajustables, facilement adaptables à la morphologie et/ou à la pathologie du patient.

Selon un premier aspect, l'invention concerne une orthèse dynamique conçue pour être montée sur une chaussure ou une semelle logée à l'intérieur d'une chaussure, soutenant le pied d'un patient et comportant :
- une embase de jambe adaptée pour entourer la jambe du patient, et
- une structure de liaison mobile reliant la chaussure ou la semelle avec l'embase et comportant un vérin élastique déporté sur l'arrière de la jambe du patient, une extension axiale dudit vérin et une articulation reliant l'extension axiale à la chaussure ou la semelle,
- l'embase comportant une coque apte à emboiter une partie de la jambe du patient et un système d'attache réglable apte à maintenir la coque autour de ladite jambe à une distance prédéfinie de la chaussure ou de la semelle.

Cette orthèse présente l'avantage d'être facilement adaptable à la morphologie et/ou la pathologie du patient.

Outre les caractéristiques qui viennent d'être évoquées dans le paragraphe précédent, l'orthèse dynamique selon un aspect de l'invention peut présenter une ou plusieurs caractéristiques complémentaires parmi les suivantes, considérées individuellement ou selon toutes les combinaisons techniquement possibles :
- la coque est fabriquée dans une résine ou un matériau thermoplastique adapté pour se déformer sous l'effet de la chaleur de sorte que ladite coque est adaptable à la morphologie de la jambe du patient.
- le système d'attache réglable comporte au moins une première courroie de fixation reliant deux bordures latérales opposées de la coque, la première courroie de fixation étant fixée directement ou indirectement sur chacune des bordures latérales.
- le système d'attache réglable comporte une première boucle de réglage fixée à une première bordure latérale de la coque et adaptée pour recevoir et verrouiller la première courroie de fixation.
- la première courroie de fixation comporte au moins une bande auto-agrippante.
- le système d'attache réglable comporte une seconde courroie de fixation, la première courroie de fixation étant positionnée en partie haute de la coque, la seconde courroie de fixation étant positionnée en partie basse de ladite coque.
- la structure de liaison mobile comporte des sangles de traction, souples et non-étirables, fixées de part et d'autre de la coque et s'étendant jusqu'au pied du patient.
- les sangles de traction sont fixées deux par deux à un même moyen de fixation et s'étendent suivant des directions non parallèles sous le pied du patient, l'une des sangles de traction assurant la traction d'une partie antérieure du pied, l'autre sangle de traction assurant une traction de la partie postérieure dudit pied.
- le moyen de fixation comporte un module d'accrochage et un module rotatif accouplés l'un à l'autre, le module d'accrochage étant accroché aux extrémités de deux sangles de traction, le module rotatif étant fixé sur la coque et autorisant une rotation suivant un axe Z.
- le module d'accrochage et le module rotatif sont accouplés par un moyen d'accouplement magnétique.
- elle comporte un carter de protection, amovible, adapté pour se fixer sur la coque et protéger le vérin élastique.

### BREVE DESCRIPTION DES FIGURES

D'autres avantages et caractéristiques de l'invention apparaîtront à la lecture de la description qui suit, illustrée par les figures dans lesquelles :
La figure 1, déjà décrite, représente une vue schématique de côté d'une orthèse dynamique selon l'état de la technique ;
La figure 2 représente une vue en perspective de la face extérieure de l'orthèse dynamique selon un mode de réalisation de l'invention ;
La figure 3 représente une vue en perspective de la face extérieure de l'orthèse dynamique selon un autre mode de réalisation de l'invention ;
La figure 4 représente des vues schématiques de différents éléments constituant l'orthèse dynamique des figures 2 et 3 ; et
La figure 5 représente une vue en perspective d'un exemple d'orthèse selon l'invention montée autour de la jambe et sur la chaussure d'un patient.

### DESCRIPTION DETAILLEE

Un exemple de réalisation d'une orthèse dynamique, facilement adaptable au patient, est décrit en détail ci-après, en référence aux dessins annexés. Cet exemple illustre les caractéristiques et avantages de l'invention. Il est toutefois rappelé que l'invention ne se limite pas à cet exemple.

Sur les figures, les éléments identiques sont repérés par des références identiques. Pour des questions de lisibilité des figures, les échelles de taille entre éléments représentés ne sont pas respectées.

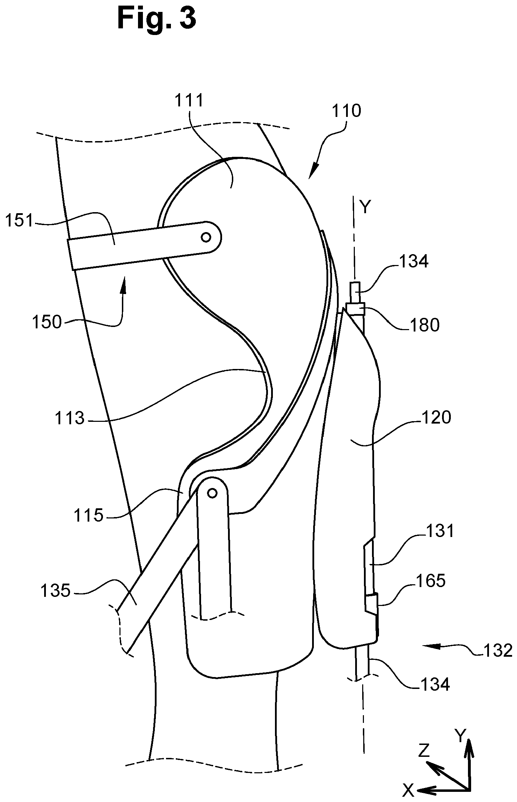

Dans la description qui va suivre, les notions de « haut » et « bas », comme par exemple la partie haute et la partie basse du vérin ou de la coque, sont définies suivant un axe Y sensiblement parallèle à l'axe de la jambe du patient (l'axe Y est représenté en figure 3), le « haut » étant considéré comme le plus proche du poplité du patient, par opposition au « bas » considéré comme le plus proche de la cheville du patient.

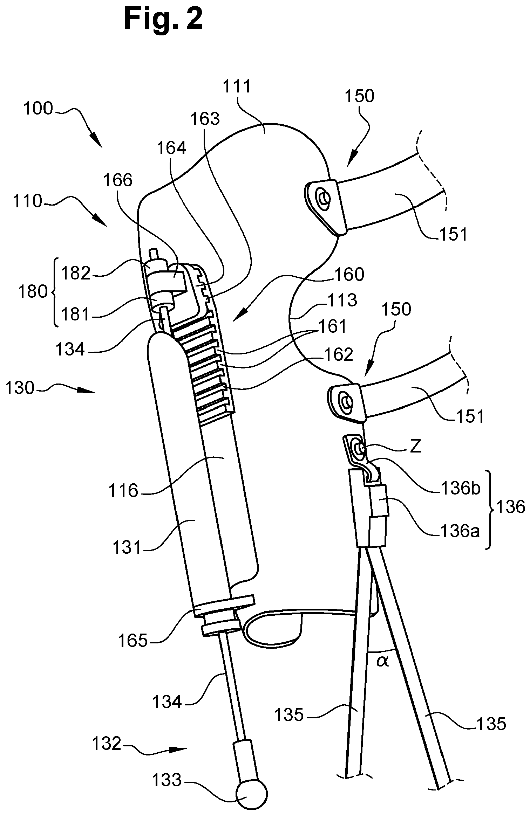

Une orthèse dynamique selon un mode de réalisation de l'invention est représentée sur les figures 2 et 3. Cette orthèse dynamique 100 est prévue pour être montée sur une chaussure soutenant le pied du patient ou une semelle adaptée, par exemple en carbone. La chaussure peut être une chaussure spécifique, adaptée à la pathologie du patient, ou une chaussure standard dans laquelle est logée une semelle spécifique adaptée à la pathologie du patient. Dans le cas d'une chaussure spécifique, l'orthèse selon l'invention est montée à l'arrière de la chaussure, sensiblement au droit du contrefort de la chaussure, par exemple au niveau de la baguette. Dans le cas d'une chaussure standard, l'orthèse selon l'invention est montée à l'arrière de la semelle, sensiblement en regard du talon. Dans la suite de l'invention, on utilisera indifféremment le terme « chaussure » pour une chaussure spécifique ou pour une semelle équipant une chaussure standard.

L'orthèse dynamique 100, appelée plus simplement orthèse, comporte une embase de jambe 110 adaptée pour entourer au moins partiellement la jambe du patient et supporter une structure de liaison mobile 130 reliant la chaussure à ladite embase. La structure de liaison mobile 130 comporte un vérin élastique 131 (appelé par la suite simplement vérin) positionné à l'arrière de la jambe du patient suivant un axe Y sensiblement parallèle à l'axe de la jambe du patient. Ce vérin élastique 131, par exemple un vérin pneumatique ou mécanique, comporte plusieurs chambres, par exemple deux ou trois, équipées de ressorts ou de lames-ressorts imbriquées les unes dans les autres, qui vont permettre de générer des forces et des vitesses modulées en fonction des mouvements de marche du patient, ces forces et vitesses modulées permettant de reproduire un effet de déroulement normal d'un pied lors d'un mouvement de marche.

La structure de liaison 130 comporte, en outre, une extension axiale 132 du vérin 131. Cette extension axiale 132 comporte une tige 134 traversant de part en part, axialement, le vérin 131 et se prolongeant jusqu'à une articulation 133 fixée à la chaussure, par exemple au niveau de la baguette de la chaussure. La tige 134 de l'extension axiale 132 est conçue pour coulisser à travers les chambres du vérin 131 et, ainsi, entraîner le pivotement de la chaussure autour de l'articulation 133. En effet, le vérin 131 est extensible suivant l'axe Y et entraîne un déplacement longitudinal de l'extension axiale 132, ce déplacement longitudinal entraînant le pivotement de la chaussure du patient autour de l'articulation de la cheville dudit patient.

Le vérin 131 peut être réglé en semi-compression lorsque la jambe du patient est perpendiculaire au pied dudit patient. Ainsi, lorsque le patient soulève son pied du sol pour faire un pas, l'extension axiale 132 se déplace vers le bas (en direction du sol) et la chaussure pivote de manière à écarter du sol la pointe avant du pied. Au contraire, lorsque l'extension axiale 132 se déplace vers le haut (en direction de la cuisse du patient), elle entraîne l'arrière de la chaussure, ce qui permet à l'avant du pied du patient de pivoter en direction du sol.

L'embase de jambe 110 comporte, selon certains modes de réalisation, une coque rigide 111 d'une forme adaptée au contour de la jambe du patient et notamment de la partie postérieure de la jambe du patient. On appelle « partie postérieure » ou « partie arrière » de la jambe, la portion de jambe qui s'étend du talon jusqu'au poplité.

La coque 111 peut, par exemple, présenter une forme sensiblement semi-ovoïde ou partiellement tubulaire, avec une face intérieure 112 creuse et arrondie et deux bordures latérales 115 s'étendant le long de la jambe du patient, symétriquement l'une et l'autre par rapport à la jambe. Les deux bordures latérales 115 peuvent prendre fin sur les côtés interne et externe de la jambe ou bien sur le devant de la jambe ou partie antérieure. La forme semi-ovoïde de la coque 111 permet d'emboiter une portion au moins de la partie postérieure de la jambe du patient, et notamment le mollet du patient.

La coque 111 peut être, par exemple, réalisée dans un matériau thermoplastique, comme du polyéthylène ou du polycarbonate, ou dans une résine, à base par exemple de carbone, ces matériaux présentant l'avantage de pouvoir être modelés lorsqu'ils sont chauffés. Ainsi, en chauffant une coque 111 en matériau thermoplastique, il est possible d'agrandir ou de rétrécir sensiblement les dimensions de la coque ou même de modifier sensiblement la forme de la coque afin de permettre une adaptation parfaite de ladite coque à la morphologie de la jambe du patient. Un thérapeute ou un fabriquant peut ainsi, par exemple, avoir plusieurs coques de dimensions différentes en stock, la coque dont les dimensions correspondent le mieux au patient pouvant ensuite être chauffée et modelée pour s'adapter parfaitement à la morphologie du patient.

Pour assurer le meilleur emboitement possible sur la jambe du patient et ainsi lui offrir le meilleur confort, la coque 111 peut comporter des échancrures latérales 113, comme représenté sur les figures 2 et 3. Ces échancrures latérales 113 peuvent être des trouées ou des découpes arrondies, par exemple en forme de C ou de C inversé, réalisées à proximité ou sur la bordure latérale 115 de la coque. Une telle échancrure 113 offre non seulement un meilleur confort au patient, mais permet également une meilleure adaptabilité à la morphologie de chaque patient.

La forme semi-ovoïde de la coque 111 confère une structure sensiblement concave à la face intérieure 112 de la coque, cette structure concave étant adaptée pour recevoir et emboiter l'arrière de la jambe du patient, et en particulier le mollet. La face intérieure 112 peut, dans certaines variantes, être recouverte d'une couche de matériau souple, comme du silicone ou un matériau textile, assurant au patient un contact plus souple et plus confortable que le matériau thermoplastique de la coque.

Comme représenté sur les figures 2 et 3, l'orthèse 100 comporte un système d'attache 150 fixé latéralement à proximité de chaque bordure latérale de la coque 111 pour permettre de maintenir ladite coque autour de la jambe du patient. Ce système d'attache 150 est réglable, de sorte à assurer un maintien facile de la coque à la jambe du patient. Ce système d'attache 150 est monté de part et d'autre de la coque, sur la face extérieure 114 de la coque, au voisinage des bordures latérales 115. Le système d'attache 150 peut comporter un unique moyen d'attache, par exemple positionné en partie haute de la coque (c'est-à-dire dans la partie de la coque la plus proche du genou du patient), comme représenté sur la figure 3. En variante, le système d'attache 150 peut comporter un double moyen d'attache, un premier moyen d'attache étant positionné en partie haute de la coque, un second moyen d'attache étant positionné en partie basse de la coque (c'est-à-dire dans une partie plus proche de la cheville du patient), comme représenté sur la figure 2. Bien entendu, le système d'attache peut comporter trois ou plus moyens d'attache, en fonction notamment des dimensions de la coque, de la distance entre les deux bordures latérales 115 de la coque, de spécificités morphologiques du patient, etc.

Selon certains modes de réalisation, chaque moyen d'attache 150 comporte deux éléments d'attache positionnés en vis-à-vis sur les deux bordures latérales 115. Les deux éléments d'attache peuvent être, par exemple, deux courroies de fixation, une courroie de fixation et une boucle, un crochet et une boucle, etc. Dans les exemples des figures 2 et 3, le moyen d'attache comporte une courroie de fixation 151 associée à une boucle de réglage de sorte que la courroie de fixation relie indirectement les deux côtés de la coque. La courroie de fixation 151 est fixée, sur un des côtés de la coque, directement au moyen d'un rivet ou de tout autre moyen de fixation directe et, sur l'autre côté de la coque, indirectement par l'intermédiaire de la boucle de réglage. La courroie de fixation 151 et la boucle de réglage sont fixées à proximité chacune d'une bordure latérale 115, par exemple à l'entrée de l'échancrure latérale 113. La courroie de fixation 151 maintient les deux côtés de la coque assemblés autour de la jambe du patient, par insertion dans la boucle de réglage.

Selon un autre exemple, le moyen d'attache 150 comporte une première courroie de fixation fixée sur un côté de la coque et une deuxième courroie de fixation fixée sur l'autre côté de la coque, les deux courroies étant maintenues ensemble au moyen d'une boucle, d'un crochet ou par agrippement.

Que le moyen d'attache comporte une seule courroie ou deux courroies de fixation, celles-ci peuvent avantageusement être des bandes auto-agrippantes telles que des bandes Velcro^{®}, conçues pour s'agripper l'une à l'autre.

Dans certains modes de réalisation, deux moyens d'attache 150 similaires sont montés sur la coque 111, l'un des moyens d'attache étant monté en partie haute de la coque, l'autre moyen d'attache étant monté en partie basse de ladite coque. Différents moyens d'attache peuvent également être montés en partie haute et en partie basse de la coque. Dans l'exemple de la figure 2, les deux moyens d'attache sont similaires, un premier moyen d'attache 150 étant monté à l'entrée des échancrures 113 des bordures latérales 115, un second moyen d'attache 150 étant monté à la sortie des échancrures 113 des bordures latérales 115.

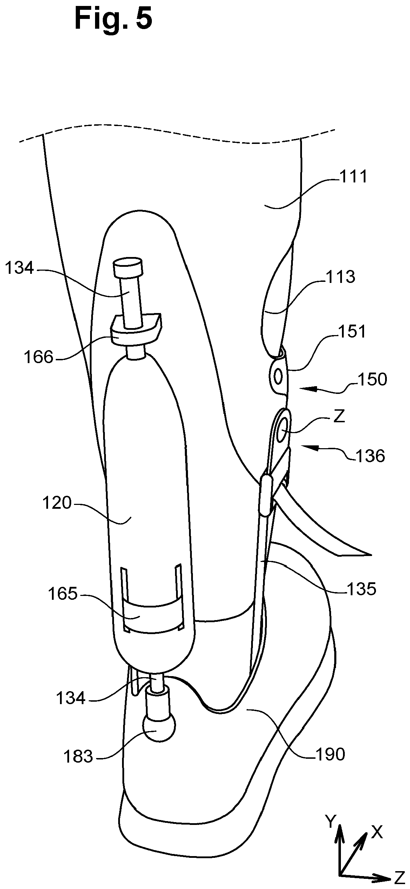

La structure de liaison mobile 130 comporte des sangles de traction 135 montées sur la coque 111 et s'étendant entre ladite coque 111 et le pied du patient. Ces sangles de traction 135 sont conçues pour être en tension pendant tous les mouvements de la marche. Chaque sangle de traction 135 est fixée d'une part sur la coque par un moyen de fixation 136 et d'autre part sur la chaussure 190, comme représenté sur la figure 5, ou sur la semelle de la chaussure, par un moyen de fixation non visible sur les figures. Lorsqu'elle est fixée sur la chaussure, l'extrémité des sangles peut passer à l'intérieur de la chaussure, par exemple entre le pied du patient et l'empeigne de la chaussure ou entre deux couches de matériaux constituant l'empeigne.

Selon un exemple, une sangle 135 peut être positionnée latéralement de chaque côté du pied, comme dans l'exemple de la figure 5, chacune des sangles 135 reliant le dessous du pied, par exemple, au niveau de la coute plantaire, et la coque 111.

Selon un autre exemple, deux sangles de traction 135 peuvent être positionnées latéralement de chaque côté du pied, l'une reliant l'avant de la voute plantaire à la coque 111, l'autre reliant le talon à ladite coque 111, comme représenté sur la figure 2. Une telle répartition des sangles de traction 135 permet une transmission optimale de la force fournie par le vérin 131. Le choix de l'emplacement des sangles sous le pied du patient peut varier et dépendre, notamment, de la pathologie du patient.

Ces sangles de traction 135 sont souples mais non-étirables ; en effet, elles sont souples afin de de pouvoir plier au cours des mouvements de marche ; elles sont non-étirables, c'est-à-dire qu'elles autorisent une déformation élastique quasiment nulle, notamment en traction longitudinale afin d'empêcher les torsions du pied. Pour cela, les sangles de traction sont fabriquées dans un matériau ayant de bonnes propriétés mécaniques en traction comme, par exemple, le Kevlar^{®}. Elles peuvent également être fabriquées sous forme de fines bandes métalliques flexibles. Les sangles de traction sont, par exemple, conçues pour présenter une résistance à la traction de l'ordre de 400Kg.

Une extrémité de chacune des sangles de traction 135 est montée mobiles en rotation autour d'un axe Z perpendiculaire à la surface de la coque 111 ; une autre extrémité desdites sangles de traction est fixée dans la chaussure. Les sangles de traction 135 ont ainsi pour fonction de gérer la dynamique et l'équilibre du pied et donc de contrôler les mouvements du pied lors de la marche en assurant une bonne supination et une bonne pronation du pied ; elles permettent notamment de limiter les angles de torsion du pied autour d'un axe X perpendiculaire à l'axe Y, qui peuvent être à l'origine d'entorses ou de fractures.

Les sangles de tractions sont montées sur la coque 111 par l'intermédiaire de moyens de fixation 136 à rotation libre qui autorisent le basculement du pied autour de la cheville, c'est-à-dire autour d'un axe Z. Chacune des sangles peut être fixée sur la coque 111 par un moyen de fixation distinct. Deux sangles de traction localisées sur un même côté du pied peuvent être fixées par un même moyen de fixation 136. Dans les exemples des figures 2 et 3, les sangles sont fixées deux par deux sur la coque, deux sangles de traction étant fixées via un même moyen de fixation 136 suivant des directions non parallèles. Dans ces exemples, les deux sangles sont assemblées sur un même module d'accrochage 136a avec un décalage angulaire α de sorte que l'une des sangles est dirigée vers la partie antérieure du pied (partie située entre les orteils et la voute plantaire) et l'autre sangle est dirigée vers la partie postérieure du pied (partie située entre la voute plantaire et le talon). Le module d'accrochage 136a est relié à la coque 111 par un module rotatif 136b lui-même fixé sur la coque. Le module d'accrochage peut être, par exemple, un boîtier serti ou riveté sur les extrémités des sangles de traction. Le module rotatif 136b peut être, par exemple, une liaison pivot. Le module d'accrochage 136a et le module rotatif 136b peuvent être reliés ensemble par tout moyen d'attache autorisant à l'ensemble un unique degré de liberté autour de l'axe Z. Dans certains modes de réalisation, le module rotatif 136b et le module d'accrochage 136a sont reliés l'un à l'autre par accouplement magnétique, par exemple au moyen d'un fermoir magnétique. En variante, ils peuvent être reliés par accouplement mécanique, par exemple au moyen de boucles rapides.

Les sangles de traction 135 ont chacune une longueur adaptée à la morphologie et/ou la pathologie du patient. Pour cela, les sangles de traction 135 sont découpées à la longueur adéquate et fixées sur la coque par les moyens de fixation 136. Dans les modes de réalisation où les moyens de fixation 136 comportent un module d'accrochage 136a et un module rotatif 136b, les sangles, après découpe, sont fixées sur le module d'accrochage de sorte que les sangles et/ou la chaussure sont interchangeables.

Dans le procédé de mise en place de l'orthèse sur le pied du patient, le vérin 131 peut être fixé dans la position adéquate pour le patient avant ou après la mise à la longueur des sangles de traction 135. Par contre, le réglage de la force du vérin, en fonction notamment du poids et de la démarche du patient, est effectué nécessairement après que les sangles de tractions 135 aient été réglées, par exemple au moyen du réglage fin 180. En effet, dans ce procédé de mise en place de l'orthèse, le praticien prend, en priorité, en compte les mouvements de pied autorisés et/ou interdits et donc les degrés de liberté autorisés avant de régler la force du vérin.

Selon l'invention, l'embase 110 comporte un système à crémaillère 160 conçu pour porter le vérin 131 et assurer une mobilité longitudinale dudit vérin par rapport à l'embase. Le réglage de la position longitudinale du vérin - c'est-à-dire le long de l'axe Y - permet d'adapter la position du vérin en fonction de la morphologie du patient, et notamment de la longueur de sa jambe. Pour une personne de grande taille, le vérin 131 est fixé sur la partie basse du système à crémaillère 160 (c'est-à-dire sur la partie du système à crémaillère la plus proche de la cheville du patient) ; pour une personne de petite taille, le vérin 131 est fixé sur la partie haute du système à crémaillère 160 (c'est-à-dire sur la partie du système à crémaillère la plus proche du poplité du patient).

Selon le mode de réalisation représenté sur la figure 2, le système à crémaillère 160 comporte une crémaillère 162 intégrée à la coque 111 de l'embase. Cette crémaillère 162 comporte une succession d'échelons ou de dents 161 formant un pignon s'étendant longitudinalement le long de l'axe Y et adapté pour emboîter une griffe de support 164 portant le vérin 131. La crémaillère 162 peut, par exemple, être moulée dans le matériau thermoplastique ou la résine formant la coque lors de la fabrication de ladite coque. La griffe de support 164 est un élément d'ancrage, par exemple en plastique ou en résine, sur lequel est fixé le vérin 131 et assurant une liaison glissière du vérin 131 sur la crémaillère 162. La griffe de support 164 comporte, sur une face, des crans 163 aptes à embrasser les échelons 161 de la crémaillère 162 et, sur une face opposée, un orifice traversant 166, ou un tunnel, apte à être traversé par la tige 134 de l'extension axiale 132. La griffe de support 164 est installée sur la face extérieure de la coque 111 et maintenue sur la crémaillère 162 au moyen d'une contre-platine 171, par exemple en métal ou en résine, installée sur la face intérieure de ladite coque 111.

Dans une variante, comme celle de la figure 2, la griffe de support 164 et la contre-platine 171 sont fixées l'une à l'autre à travers la coque 111, au moyen d'un système de fixation du type vis ou rivet. Dans une autre variante, comme celle de la figure 5, la coque 111 comporte des orifices longitudinaux 116, de part et d'autre de la crémaillère 162, pour permettre une liaison directe entre la contre-platine 171 et la griffe de support 164 au moyen d'un système de fixation comme des vis, rivets, clips, etc. Quelle que soit le mode de fixation de la griffe de support 164, l'imbrication de la griffe de support 164 et de la crémaillère 162 permet un déplacement du vérin 131 le long de la crémaillère 162 et un maintien de ladite griffe de support à l'emplacement choisi, le vérin 131 étant continuellement positionné le long de l'axe Y.

La mobilité de la griffe de support 164 par rapport à la coque 111, c'est-à-dire la possibilité de déplacer ladite griffe de support 164 sur la crémaillère 162, permet d'ajuster la position du vérin 131 par rapport à la chaussure du patient, en fonction de la morphologie du patient. Une fois la position définie, la griffe de support 164 est maintenue en position sur la coque 111 au moyen de la contre-platine 171, montée de préférence en intérieur de coque. Un exemple d'une contre-platine 171 fixée à la griffe de support 164, par des vis ou des rivets 172, est représenté sur la figure 5. Cette figure 5 montre la face intérieure 112 de la coque 111 avec la crémaillère 162 et la contre-platine 171.

Ainsi, la partie haute du vérin 131 est fixée, via la griffe de support 164 et la tige 134 de l'extension axiale 132, sur la coque 111, le long de la crémaillère 162. Le vérin 131 est également fixé, en partie basse, à la coque 111 au moyen d'une attache 165 en forme de U, de type cavalier. Le cavalier 165 peut être introduit directement dans des encoches réalisées dans la coque 111, comme représenté sur la figure 2. Il peut également être clipsé sur un support à encoches faisant partie intégrante de la coque 111 ou monté solidaire de ladite coque. Dans cette dernière variante, plusieurs supports à encoches peuvent être prévus sur la coque 111 de sorte à permettre plusieurs niveaux de fixation basse du vérin 131. Les dimensions et l'emplacement du cavalier 165, et plus généralement de l'attache en forme de U, ont un effet sur le mouvement d'avant en arrière autorisé au vérin, c'est-à-dire le débattement autorisé au vérin 131 et à l'extension axiale 132 suivant l'axe X.

Le vérin 131 est donc rendu solidaire de la coque 111 au moyen de deux éléments de fixation : la griffe de support 164 qui solidarise le vérin 131 sur la crémaillère 162 et le cavalier 165 qui solidarise le vérin 131 directement sur la coque 111. En particulier, la griffe de support 165 est montée en partie haute ou en milieu d'embase 110 pour maintenir la partie haute du vérin 131 et le cavalier 165 est monté en partie basse de l'embase 110 pour fixer la partie basse du vérin 131. Le vérin 131 est ainsi maintenu fixement à l'emplacement choisi sur la crémaillère, le long de l'axe Y, tout en lui permettant d'être mobile via son extension axiale 132 et d'éviter toutes contraintes de cisaillement.

Le choix de l'emplacement des éléments de fixation (griffe de support et cavalier) peut avoir un effet sur la longueur de déplacement de la tige 134 et sur la force et/ou la vitesse générée par le vérin. En particulier, en fonction du montage de la griffe de support 164, la tige 134 de l'extension axiale 132 peut s'étendre plus ou moins loin hors de la coque 111 et être plus ou moins freinée dans son coulissement, ce qui a un effet sur la force et la vitesse de déplacement de ladite tige 134. Bien entendu, d'autres modes de réalisation des éléments de fixation peuvent être envisagés dès lors qu'ils sont aptes à rendre le vérin 131 solidaire de la crémaillère 162 et/ou de la coque 111 et qu'ils permettent un ajustement de la longueur, de la force et de la vitesse de déplacement du vérin 131.

Dans certains modes de réalisation et notamment dans celui de la figure 2, l'orthèse comporte un dispositif de réglage fin 180 monté sur la griffe de support 164 pour régler la force du vérin 131. Ce dispositif de réglage fin 180 peut comporter deux écrous 181, 182 montés autour de la tige 134 qui, au moins à son extrémité haute, peut être filetée. Dans l'exemple de la figure 2, la partie filetée de la tige 134 de vérin traverse un premier écrou 181 et un second écrou 182, alignés longitudinalement et positionnés de part et d'autre du tunnel 166. Les deux écrous 181, 182 sont donc alignés suivant l'axe Y avec le vérin 131 de sorte que tout déplacement de l'un au moins des deux écrous entraîne une modification de la compression interne du vérin et donc de la force fournie par ledit vérin.

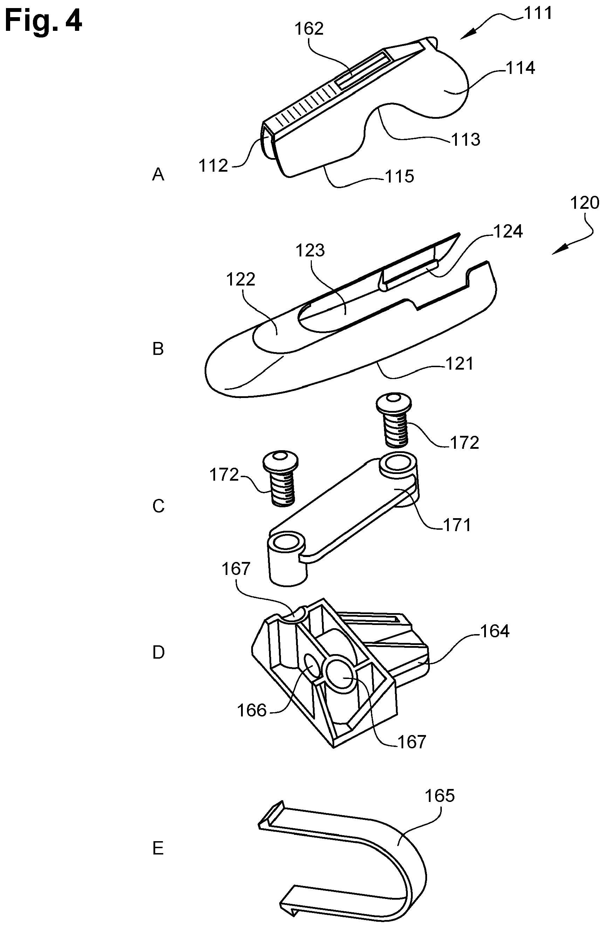

La figure 4 représente une vue distincte de différents éléments de l'orthèse selon l'invention. La vue A représente un exemple d'une coque 111 telle que décrite précédemment dans laquelle est intégrée la crémaillère 162 ; la vue B représente un exemple d'un carter 120 qui va être décrit ultérieurement ; la vue C représente un exemple d'une contre-platine 171 adaptée pour recevoir des vis ou rivets 172 ; la vue D représente un exemple de la griffe de support 164 décrite précédemment et dont les orifices 167 sont prévus pour recevoir les vis ou rivets 172; la vue E représente un exemple de cavalier 165 dont les extrémités sont adaptées pour se clipser dans des encoches.

Le système à crémaillère 160 qui vient d'être décrit permet d'adapter facilement la position et la force du vérin à la morphologie et/ou la pathologie du patient. Lors de la première installation de l'orthèse 100 sur la jambe du patient, le thérapeute ou le fabriquant veille à positionner le vérin, le long de l'axe Y, à l'emplacement le mieux adapté à la morphologie du patient et/ou à sa pathologie afin que, d'une part, la distance entre la chaussure et le vérin et, d'autre part, la force et la vitesse de déplacement du vérin soient optimales pour le patient. Pour cela, le thérapeute ou le fabriquant déplace la griffe de support 164 sur la crémaillère 162, le long de l'axe Y, jusqu'à l'emplacement choisi puis fixe ladite griffe de support 164 sur la coque au moyen de la contre-platine 171. Un ajustement plus précis de la force et/ou de la vitesse de déplacement du vérin peut ensuite être défini par rotation d'un ou des deux écrous 181, 182 sur la tige du vérin.

Dans certains modes de réalisation, le vérin 131 est protégé par un carter 120 monté de façon amovible sur la coque 111 de sorte à entourer au moins en partie le vérin. Ce carter 120, dont un exemple est représenté sur le dessin B de la figure 4, forme une coquille, ouverte sur la face 121 en regard de la coque 111 et destinée à recevoir le vérin. La face opposée 122 peut être fermée ou bien comporter un orifice 123 permettant un accès visuel au vérin 131. Les parois latérales internes du carter peuvent comporter des encoches de fixation 124 adaptées pour recevoir des ailettes en saillie sur le vérin 131. Ces encoches 124, par exemple au nombre de deux, peuvent être logées directement dans les parois internes du carter. Elles peuvent, en variante, être logées chacune dans une structure en saillie sur les parois internes dudit carter, ces structures en saillie formant une butée de positionnement du carter sur le vérin 131. Le vérin 131 comporte, sur sa surface externe, des ailettes, par exemple au nombre de deux, de forme adaptée pour s'emmancher dans les encoches de fixation 124. Ce carter 120, facile à monter et à démonter, permet de protéger le vérin des éventuels chocs et d'empêcher les vêtements du patient de s'accrocher dans le vérin.

Bien que décrit à travers un certain nombre d'exemples, variantes et modes de réalisation, l'orthèse dynamique selon l'invention comprend divers variantes, modifications et perfectionnements qui apparaîtront de façon évidente à l'homme du métier, étant entendu que ces variantes, modifications et perfectionnements font partie de la portée de l'invention.

## Revendications

1. Orthèse dynamique (100) conçue pour être montée sur une chaussure ou une semelle logée à l'intérieur d'une chaussure, soutenant le pied d'un patient et comportant :
∘ une embase de jambe (110) adaptée pour entourer la jambe du patient,
et
∘ une structure de liaison mobile (130), reliant la chaussure ou la semelle avec l'embase et comportant un vérin élastique (131) déporté sur l'arrière de la jambe du patient, une extension axiale (132) dudit vérin et une articulation (133) reliant l'extension axiale à la chaussure ou la semelle, l'embase (110) comportant une coque (111) apte à emboiter une partie de la jambe du patient et un système d'attache réglable (150) apte à maintenir la coque autour de ladite jambe à une distance prédéfinie de la chaussure ou de la semelle.

2. Orthèse dynamique selon la revendication 1, **caractérisée en ce que** la coque (111) est fabriquée dans une résine ou un matériau thermoplastique adapté pour se déformer sous l'effet de la chaleur de sorte que ladite coque soit adaptable à la morphologie de la jambe du patient.

3. Orthèse dynamique selon la revendication 1 ou 2, **caractérisée en ce que** le système d'attache réglable (150) comporte au moins une première courroie de fixation (151) reliant deux bordures latérales (115) opposées de la coque, la première courroie de fixation étant fixée directement ou indirectement sur chacune des bordures latérales.

4. Orthèse dynamique selon la revendication 3, **caractérisée en ce que** le système d'attache réglable (150) comporte une première boucle de réglage fixée à une première bordure latérale (115) de la coque et adaptée pour recevoir et bloquer la première courroie de fixation (151).

5. Orthèse dynamique selon la revendication 3 ou 4, **caractérisée en ce que** la première courroie de fixation (151) comporte au moins une bande auto-agrippante.

6. Orthèse dynamique selon l'une quelconque des revendications 3 à 5, **caractérisée en ce que** le système d'attache réglable (150) comporte une seconde courroie de fixation (151), la première courroie de fixation étant positionnée en partie haute de la coque, la seconde courroie de fixation étant positionnée en partie basse de ladite coque.

7. Orthèse dynamique selon l'une quelconque des revendications 1 à 6, **caractérisée en ce que** la structure de liaison mobile (130) comporte des sangles de traction (135), souples et non-étirables, fixées de part et d'autre de la coque et s'étendant jusqu'au pied du patient.

8. Orthèse dynamique selon la revendication 7, **caractérisée en ce que** les sangles de traction (135) sont fixées deux par deux à un même moyen de fixation (136) et s'étendent suivant des directions non parallèles sous le pied du patient, l'une des sangles de traction assurant la traction d'une partie antérieure du pied, l'autre sangle de traction assurant une traction de la partie postérieure dudit pied.

9. Orthèse dynamique selon la revendication 8, **caractérisée en ce que** le moyen de fixation (136) comporte un module d'accrochage (136a) et un module rotatif (136b) accouplés l'un à l'autre, le module d'accrochage étant accroché aux extrémités de deux sangles de traction, le module rotatif étant fixé sur la coque et autorisant une rotation suivant un axe (Z).

10. Orthèse dynamique selon la revendication 9, **caractérisée en ce que** le module d'accrochage (136a) et le module rotatif (136b) sont accouplés par un moyen d'accouplement magnétique.

11. Orthèse dynamique selon l'une quelconque des revendications 1 à 10, **caractérisée en ce qu'**elle comporte un carter de protection (120), amovible, adapté pour se fixer sur la coque (111) et protéger le vérin élastique (131).
